# EUROPEAN PATENT SPECIFICATION

(11) **EP 2 705 867 B1**
(45) Date of publication and mention of the grant of the patent: **21.09.2016**
(21) Application number: 13182431.0
(22) Date of filing: 30.08.2013
(51) Int. Cl.: A61M 13/00, A61B 1/015, A61B 1/00, A61B 1/06, A61M 5/00

(54) **Gas-supply system**
Gasversorgungssystem
Système d'alimentation en gaz

(30) Priority: 06.09.2012 JP 2012196420
(43) Date of publication of application: 12.03.2014
(73) Proprietor: Fujifilm Corporation, Minato-ku Tokyo 106-8620 (JP)
(72) Inventor: Torisawa, Nobuyuki, Kanagawa, 258-8538 (JP); Serizawa, Mitsuhiko, Kanagawa, 258-8538 (JP); Hayashi, Kentaro, Kanagawa, 258-8538 (JP)
(74) Representative: Klunker . Schmitt-Nilson . Hirsch

(56) References cited:
- WO-A2-01/80923
- US-A- 3 674 010
- US-A1- 2006 030 751
- US-A1- 2007 255 106
- US-B1- 8 216 159

## Description

### 1. Field of the invention

The present invention relates to a gas-supply system according to the preamble of claim 1, and particularly, a gas-supply system that supplies predetermined gas (for example, carbon dioxide gas) into a body cavity of a subject.

### 2. Description of the Related Art

When inspection or medical treatment is performed using an endoscope, in order to secure the visual field of the endoscope or secure a region where a treatment tool is operated, gas is supplied into a body cavity from a gas supply conduit provided in the endoscope. As the gas to be supplied into the body cavity, air is mainly used in the related art, but carbon dioxide gas (CO₂ gas) has been used in recent years. Since carbon dioxide gas has favorable bioabsorbability, there is little damage to the subject. For this reason, there is a tendency for carbon dioxide gas to be used as a gas supply source.

When carbon dioxide gas is supplied into the body cavity, a gas supply device to which a gas cylinder filled with the carbon dioxide gas is attached is used. The gas supply device is detachably connected to a gas supply conduit of the endoscope, and the carbon dioxide gas from the carbon dioxide gas cylinder is pressure-reduced and supplied.

For example, a gas-supply system described in JP 2006-130077A automatically supplies the carbon dioxide gas into the body cavity on the basis of the detection result of a pressure sensor that detects the pressure within the body cavity so that the pressure within the body cavity becomes a set pressure. This enables the pressure within the body cavity to be stably controlled to a desired state without requiring an operator's complicated operation.

However, in the gas-supply system described in JP 2006-130077A, a front panel of an air supply device should be checked in order to check whether or not the carbon dioxide gas is automatically supplied into the body cavity. Generally, the air supply device is installed in a cart together with other instruments, and these are arranged at a position relatively distant from the operator. For this reason, when the operator is operating the endoscope, it is difficult to directly check whether or not the carbon dioxide gas is automatically supplied into the body cavity.

In accordance with the preamble of claim 1, US 3 674 010 A discloses a gas supply system in which the gas supply pipe has incorporated a manually operable valve. After an inflatable tube has been inserted into a body cavity, this valve is opened by an operator. When a predetermined pressure has been reached, the pressure detecting means of the system is activated to stop the supply of gas by closing an electromagnetic valve. This valve is opened again when pressure detecting means detects that the pressure has fallen below said predetermined value. After the operator has opened the manually operated valve and the gas supply has been stopped automatically, the operator may use the endoscope including the inflatable tube after glancing on a scale of a pressure gauge disposed in the valve supply pipe between the pressure detecting means and the manually operable valve.

WO 01/80923 A2 discloses a balloon member inserted into a gas supply pipe, wherein an inflation amount of the balloon member changes according to the pressure within the pipe.

US 8 216 159 discloses a gas leaking testing device in which a plunger is shifted according to the pressure within an interior space of the device. An external pin connected to the plunger indicates a pressure amount.

Further prior art is shown in US 2006/030751 A1 (pressure indication on a indicator panel) and US 2007/255106 A1 (pressure indication related to pressure within a body cavity using a controller).

The invention has been made in view of such a situation, and an object thereof is to provide a gas-supply system in which it can be easily grasped that gas is automatically supplied into a body cavity.

In order to achieve the above object, the gas-supply system related to the invention is a gas-supply system that supplies gas into a body cavity of a subject. The gas-supply system includes the features of claim 1.

According to the invention, when an operator is operating the endoscope, it is possible to easily grasp the supply state of the gas by the automatic gas supply means simply by checking the notifying means provided in the gas supply pipe without turning his/her eyes.
This can reduce the operator's operation burden and can improve convenience.

In the invention, the gas-supply system further includes control means that outputs a control signal showing whether or not the supply of the gas by the automatic gas supply means is performed, and the notifying means is a light emitter capable of emitting light according to a control signal output from the control means. According to this aspect, since the light emitter provided in the gas supply pipe emits light according to whether or not the supply of the gas by the automatic gas supply means is performed, it is possible to easily grasp the supply state of the gas by the automatic gas supply means.

The gas-supply system further includes a light source capable of emitting light according to a control signal output from the control means, and the light emitter is a side face leakage type optical fiber on which the light emitted by the light source is incident and that transmits the light while leaking light to a side face.

According to the invention, when an operator is operating the endoscope, it is possible to easily grasp the supply state of the gas by the automatic gas supply means simply by checking the notifying means provided in the gas supply pipe without turning his/her eyes. This can reduce an operator's operation burden and can improve convenience.

### BRIEF DESCRIPTION OF THE DRAWINGS

Fig. 1 is an overall configuration view showing the schematic configuration of an endoscope system in a first embodiment.
Fig. 2 is a perspective view showing a tip portion of an insertion section of the endoscope.
Fig. 3 is a configuration view schematically showing the conduit configuration of the endoscope.
Fig. 4 is a block diagram showing the configuration of a gas supply device.
Fig. 5 is a schematic view schematically showing the surroundings of a connection between the gas supply device and a gas supply pipe.
Fig. 6 is a schematic view showing another configuration example (first modification example) of the gas supply pipe.
Fig. 7 is a schematic view showing still another configuration example (second modification example) of the gas supply pipe.
Fig. 8 is a schematic view showing a still further configuration example (third modification example) of the gas supply pipe.
Fig. 9 is a flowchart view showing an operation procedure of the gas supply device.
Fig. 10 is a flowchart view showing an example of automatic gas supply control.
Fig. 11 is a graph schematically showing the state of changes in detected flow rate before and after recovery control is executed.
Fig. 12 is a flowchart view showing an example of LED lighting control.
Fig. 13 is a view showing the correspondence relationship between the opened/closed state of first and second electromagnetic valves and the lighting state of LEDs.
Fig. 14 is a view showing an example of LED lighting control when a combination of a green LED and a red LED is used.
Fig. 15 is a view showing an example of the LED lighting control according to body cavity pressure.
Fig. 16 is a block diagram showing the configuration of a gas supply device.
Fig. 17 is a block diagram showing the configuration of a gas supply device.
Fig. 18 is a configuration view schematically showing the conduit configuration of an endoscope.
Fig. 19 is a schematic cross-sectional view showing a configuration example of a gas supply pipe.
Fig. 20 is a schematic view showing another configuration example of the gas supply pipe.
Fig. 21 is a schematic view showing still another configuration example of the gas supply pipe.
Fig. 22 is a configuration view schematically showing the conduit configuration of an endoscope.
Fig. 23 is a block diagram showing the internal configuration of a gas supply device.
Fig. 24 is an overall configuration view showing the schematic configuration of an endoscope system.
Fig. 25 is a conduit configuration view showing the internal configuration of an endoscope.

### DESCRIPTION OF THE PREFERRED EMBODIMENTS

A preferred embodiment of the invention will be described below in detail according to the accompanying drawings.

### First Embodiment

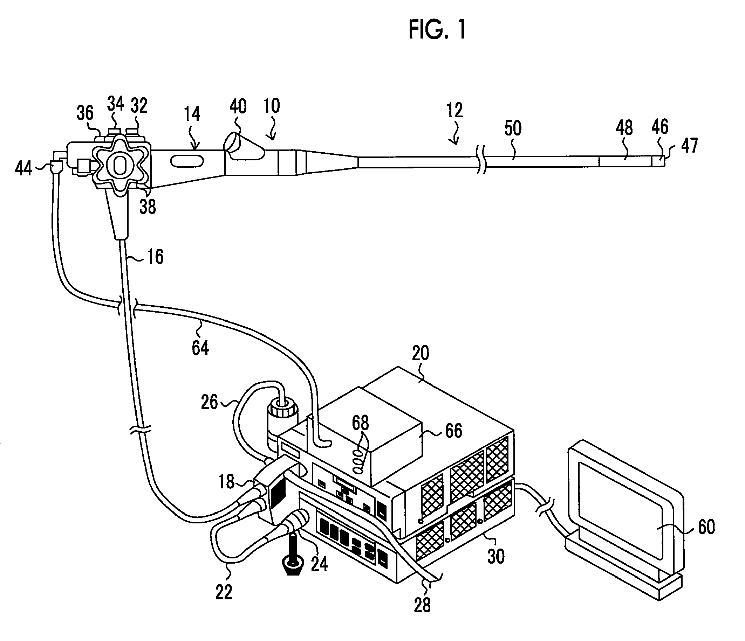

Fig. 1 is an overall configuration view showing the schematic configuration of an endoscope system to which the invention is applied. The endoscope system shown in Fig. 1 is mainly constituted of an endoscope 10, a light source device 20, a processor 30, and a gas supply device 66.

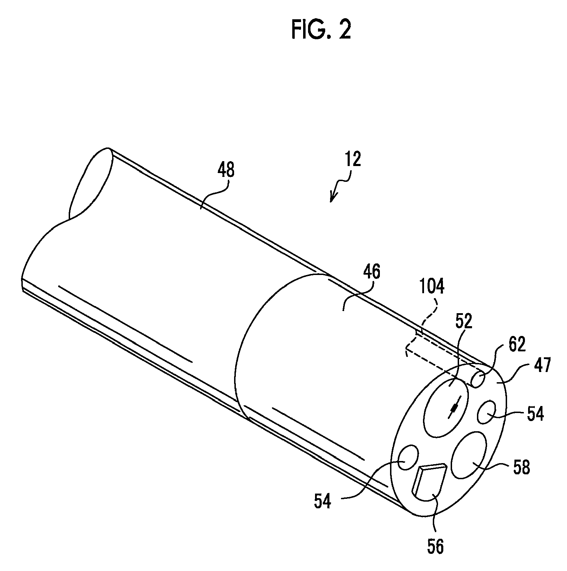

The endoscope 10 includes an insertion section 12 to be inserted into a body cavity, and a proximal operating section 14 connected to the insertion section 12. A universal cable 16 is connected to the proximal operating section 14, and an LG connector 18 is provided at the tip of the universal cable 16. By detachably coupling the LG connector 18 to the light source device 20, illumination light can be transmitted to illumination optical systems 54 (refer to Fig. 2) to be described below. Additionally, an electric connector 24 is connected to the LG connector 18 via a cable 22, and the electric connector 24 is detachably coupled to the processor 30. In addition, a gas and water supplying tube 26 and a suctioning tube 28 are connected to the LG connector 18.

In the proximal operating section 14, a gas and water supply button 32, a suction button 34, and a shutter button 36 are arranged in parallel, and a pair of angle knobs 38 and 38 and a forceps insertion portion 40 are provided. Additionally, the proximal operating section 14 is provided with a gas supply port 44 for supplying carbon dioxide gas into a body cavity.

On the other hand, the insertion section 12 includes a tip portion 46, a bending portion 48, and a soft portion 50, and the bending portion 48 is remotely operated so as to be bent by rotating the pair of angle knobs 38 and 38 provided at the proximal operating section 14. This enables a tip face 47 of the tip portion 46 to be directed in a desired direction.

As shown in Fig. 2, the tip face 47 of the tip portion 46 is provided with an observation optical system 52, the illumination optical systems 54 and 54, a gas and water supply nozzle 56, and a forceps port 58. A CCD (not shown) is disposed behind the observation optical system 52, and a signal cable is connected to a substrate that supports the CCD. The signal cable is inserted through the insertion section 12, the proximal operating section 14, and the universal cable 16 of Fig. 1, extends up to the electric connector 24, and is connected to the processor 30. Accordingly, an observation image imported by the observation optical system 52 of Fig. 2 is formed on a light-receiving surface of the CCD, is converted into an electrical signal, and is output to the processor 30 of Fig. 1 via the signal cable, and this electrical signal is converted into a video signal. This allows the observation image to be displayed on the monitor 60 connected to the processor 30.

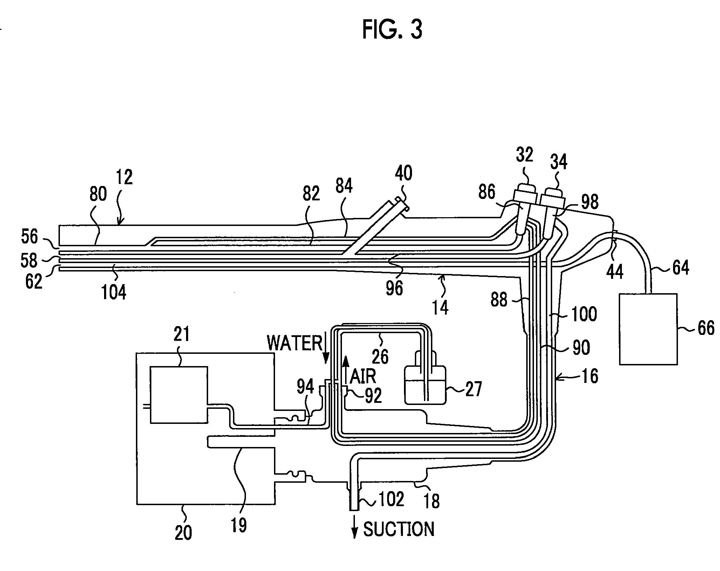

An emission end of a light guide (not shown) is disposed behind the illumination optical systems 54 and 54 of Fig. 2. The light guide is inserted through the insertion section 12, the proximal operating section 14, and the universal cable 16 of Fig. 1. An incidence end of the light guide is disposed at a light guide rod (refer to Fig. 3) 19 of the LG connector 18. Accordingly, by coupling the light guide rod 19 of the LG connector 18 to the light source device 20, the illumination light radiated from the light source device 20 is transmitted to the illumination optical systems 54 and 54 via the light guide and is radiated from the illumination optical systems 54 and 54.

Fig. 3 is a configuration view schematically showing the conduit configuration of the endoscope 10. As shown in Fig. 3, a gas and water supply tube 80 is connected to the gas and water supply nozzle 56. The gas and water supply tube 80 branches into a gas supply tube 82 and a water supply tube 84 that are respectively connected to a valve 86 disposed at the proximal operating section 14. An air supply tube 88 and a water supply tube 90 are connected to the valve 86, and the gas and water supply button 32 is attached to the valve. As the gas supply tube 82 and the air supply tube 88 communicate with each other in a state where the gas and water supply button 32 protrudes, and the gas and water supply button 32 is operated by being pressing, the water supply tube 84 and the water supply tube 90 communicate with each other. The gas and water supply button 32 is formed with a vent hole (not shown), and the air supply tube 88 communicates with the external air via the vent hole.

The air supply tube 88 and the water supply tube 90 are inserted through the universal cable 16, and extend up to a water supply connector 92 of the LG connector 18. The tube 26 is detachably connected to the water supply connector 92, and the tip of the tube 26 is coupled to a water storage tank 27. The water supply tube 90 communicates with the water storage tank 27 below its liquid level, and the air supply tube 88 communicates with the water storage tank above its liquid level.

An air tube 94 is connected to the water supply connector 92, and the air tube 94 communicates with the air supply tube 88. Additionally, the air tube 94 communicates with an air pump 21 within the light source device 20 coupling the LG connector 18 to the light source device 20. Accordingly, if the air pump 21 is driven to supply air, air is supplied to the air supply tube 88 via the air tube 94. This air escapes to the external air via a vent hole (not shown) when the gas and water supply button 32 is not operated. Thus, as an operator blocks the vent hole, the air of the air supply tube 88 is supplied to the gas supply tube 82, and air is jetted from the gas and water supply nozzle 56. Additionally, if the gas and water supply button 32 is operated by being pressing, the air supply tube 88 and the gas supply tube 82 are cut off. Therefore, the air supplied to the air tube 94 is supplied to above the liquid level of the water storage tank 27. This increases the internal pressure of the water storage tank 27 so as to allow water to be supplied to the water supply tube 90. Then, water is jetted from the gas and water supply nozzle 56 via the water supply tube 84. As water or air is jetted from the gas and water supply nozzle 56 in this way and is blown against the observation optical system 52, the observation optical system 52 is cleaned.

On the other hand, a forceps tube 96 is connected to the forceps port 58. The forceps tube 96 branches and communicates with the forceps insertion portion 40 and a valve 98. Hence, by inserting a treatment tool, such as forceps, from the forceps insertion portion 40, the treatment tool can be led out from the forceps port 58. A suction tube 100 is connected to the valve 98, and the suction button 34 is attached to the valve. The suction tube 100 communicates with the external air in a state where the suction button 34 has protruded, and the suction tube 100 and the forceps tube 96 are connected together by operating suction button 34 being operated by being pressing. The suction tube 100 extends up to a suction connector 102 of the LG connector 18, and communicates with a suction device (not shown) by connecting the tube 28 (refer to Fig. 1) to the suction connector 102. Accordingly, a lesion or the like can be suctioned from the forceps port 58 by operating the suction button 34 by being pressing in a state where the suction device is driven.

The tip face 47 of the tip portion 46 is formed with a gas jet port 62. A gas tube 104 is connected to the gas jet port 62. The gas tube 104 is connected to the gas supply port 44 disposed at the proximal operating section 14. One end of a gas supply pipe 64 is detachably connected to the gas supply port 44, and the other end of the gas supply pipe 64 is coupled to the gas supply device 66. A gas-supply system is constituted of the gas supply pipe 64 and gas supply device 66. By supplying carbon dioxide gas from the gas supply device 66, the carbon dioxide gas is jetted from the gas jet port 62 via the gas supply port 44 and the gas tube 104, and the inside of a body cavity can be swollen with the carbon dioxide gas.

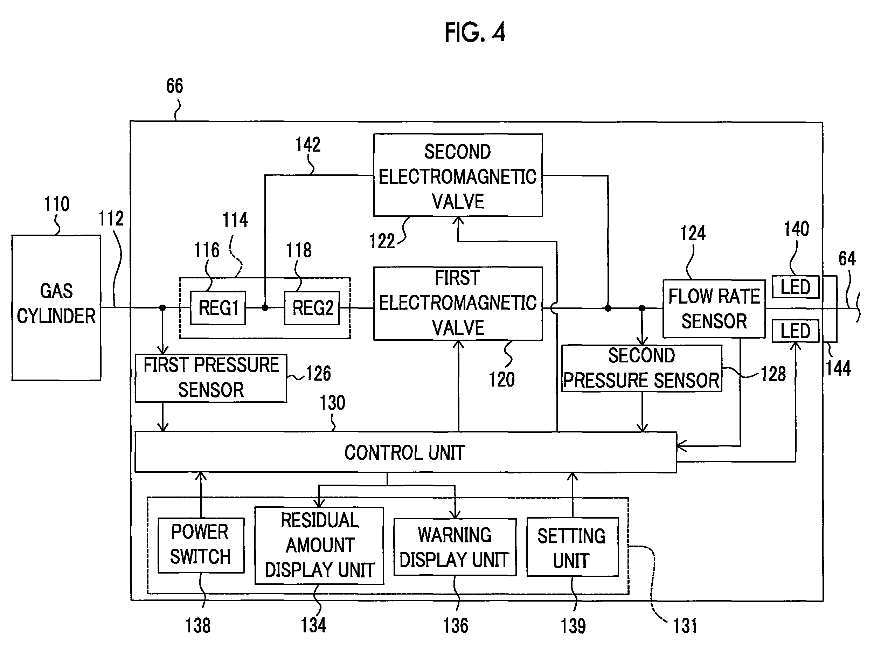

Fig. 4 is a block diagram showing the configuration of the gas supply device 66. As shown in Fig. 4, the gas supply device 66 is configured to include a pressure-reducing mechanism 114, electromagnetic valves 120 and 122, a flow rate sensor 124, pressure sensors 126 and 128, a control unit 130, an operation panel 131, and an LED 140.

A carbon dioxide cylinder 110 is detachably coupled to the gas supply device 66 via a high-pressure hose 112. Inside the gas supply device 66, the pressure-reducing mechanism 114, the first electromagnetic valve 120, and the flow rate sensor 124 are connected in series sequentially from the carbon dioxide cylinder 110 side, and the carbon dioxide gas pressure-reduced to predetermined pressure via these respective parts is supplied to an automatic gas supply connector 144.

The pressure-reducing mechanism 114 is constituted of two regulators (pressure-reducing valves) 116 and 118 that are arranged in series. The regulators 116 and 118 gradually reduce the pressure of the carbon dioxide gas supplied from the carbon dioxide cylinder 110 to suitable pressure. For example, in the first regulator 116, the pressure of the carbon dioxide gas from the carbon dioxide cylinder 110 is reduced from 10 MPa to 0.3 MPa. Additionally, in the second regulator 118, the pressure of the carbon dioxide gas reduced by the first regulator 116 is reduced from 0.3 MPa to 0.05 MPa.

The first electromagnetic valve 120 operates to open and close on the basis of a control signal output from the control unit 130, and adjusts the flow rate of carbon dioxide gas supplied into a body cavity. As the first electromagnetic valve 120, a flow rate control valve (electromagnetism proportionality valve) capable of controlling a flow rate in proportion to a control signal (current value) is preferably used. By using a flow rate control valve, it is possible to control the flow rate of carbon dioxide gas supplied into a body cavity with high precision compared to a case where a switching valve capable of being fully opened or fully closed only is used.

The flow rate sensor 124 is disposed on an outlet side of the first electromagnetic valve 120 to detect the flow rate of carbon dioxide gas supplied into a body cavity via the gas supply pipe 64 and output the detection result to the control unit 130.

The first pressure sensor 126 is connected between the carbon dioxide cylinder 110 and the pressure-reducing mechanism 114 to detect the pressure of carbon dioxide gas from the carbon dioxide cylinder 110 and output the detection result to the control unit 130.

The second pressure sensor 128 (pressure detecting means) is connected between the first electromagnetic valve 120 and the flow rate sensor 124 to detect the pressure within a body cavity via the gas supply pipe 64 or the gas tube 104 and outputs the detection result to the control unit 130.

One end of a bypass conduit 142 is connected between the first regulator 116 and the second regulator 118. The other end of the bypass conduit 142 is connected to an outlet side of the electromagnetic valve 120. This enables the carbon dioxide gas pressure-reduced by the first regulator 116 to be led to the gas supply pipe 64 without passing through the second regulator 118 and the first electromagnetic valve 120.

The second electromagnetic valve 122 is disposed at the bypass conduit 142, and operates to open and close on the basis of the control signal output from the control unit 130. As the second electromagnetic valve 122, a normally closed type is preferably used. This brings the bypass conduit 142 into an always cut-off state while gas supply of carbon dioxide gas is performed by automatic gas supply control to be described below, and can reliably prevent the outflow of the carbon dioxide gas via the bypass conduit 142.

The operation panel 131 is provided with a residual amount display unit 134, a warning display unit 136, a power switch 138, and a setting unit 139, and these respective parts are connected to the control unit 130. The setting unit 139 is provided with an operation button for inputting the set pressure within a body cavity, and if the set pressure within the body cavity is input by the operation of the operator, an input signal is output to the control unit 130.

The control unit 130 performs the overall control of the gas supply device 66, and is configured to include a CPU and a memory (all are not shown), or the like. A control program and various setting information (for example, the set pressure within the body cavity input by the setting unit 139) for operating the gas supply device 66 are stored in the memory. In addition, automatic gas supply means is constituted of the control unit 130, the first electromagnetic valve 120, and the pressure-reducing mechanism 114.

Additionally, the control unit 130 displays the residual amount of carbon dioxide gas in the carbon dioxide cylinder 110 on the residual amount display unit 134 on the basis of the pressure detected by the first pressure sensor 126. Additionally, the control unit 130 makes the warning display unit 136 display a warning and generate an alarm if the residual amount of carbon dioxide gas becomes equal to or lower than a predetermined level. This enables the carbon dioxide cylinder 110 to be replaced by a new one before the residual amount of the carbon dioxide gas is exhausted.

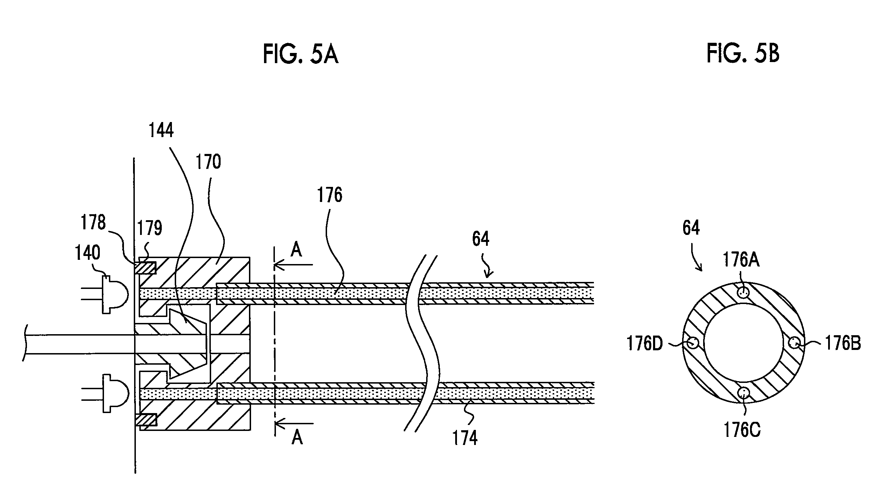

A plurality of the LEDs 140 are provided at a position adjacent to the automatic gas supply connector 144 to which the tip of the gas supply pipe 64 is detachably coupled. In the present example, four LEDs 140 are provided (two LEDs are shown in Figs. 4 and 5). Additionally, LEDs having all the same colors (for example, green, red, or the like) are used as the respective LEDs 140. The lighting state (ON/Blinking/OFF) of the respective LEDs 140, changes on the basis of a control signal output from the control unit 130.

Fig. 5 is a schematic view schematically showing the surroundings of a connection between the gas supply device 66 and the gas supply pipe 64. Fig. 5A is a side cross-sectional view, and Fig. 5B is a front cross-sectional view (cross-sectional view taken along line A-A of Fig. 5A) of the gas supply pipe 64.

As shown in Fig. 5, the gas supply pipe 64 includes a transparent tube 174 having a light-transmissive side wall portion, and a plurality of side face light leakage type optical fibers 176 (notifying means) are buried in the side wall portion along an axial direction. In addition, although the gas supply pipe 64 is provided with four side face light leakage type optical fibers 176A to 176D corresponding to the number of the LEDs 140, the number of the LEDs 140 or the side face light leakage type optical fibers 176 is not particularly limited. The tip of the gas supply pipe 64 is provided with a connection connector 170, the respective side face light leakage type optical fibers 176 extend up to the connection connector 170, and the end faces of the respective side face light leakage type optical fibers 176 are exposed to positions that face the LEDs 140, respectively. If the LEDs 140 are made to be turned on in a state where the connection connector 170 of the gas supply pipe 64 is coupled to the automatic gas supply connector 144, since the light from the LEDs 140 is incident from the end faces of the side face light leakage type optical fibers 176 and the light is transmitted while leaking light to a side face, the gas supply pipe 64 functions as a linear light emitter. Additionally, since the side face light leakage type optical fibers 176 have high flexibility (soft flexibility), and can be bent and used in arbitrary directions, the handleability of the gas supply pipe 64 is also excellent.

Additionally, in order to make the gas supply pipe 64 emit light, when the connection connector 170 of the gas supply pipe 64 is coupled to the automatic gas supply connector 144, it is necessary to align the end faces of the side face light leakage type optical fibers 176 and the LEDs 140 so as to face each other. In the present embodiment, in order to facilitate the alignment of them, as position regulating means, the wall surface of the gas supply device 66 around the automatic gas supply connector 144 is provided with a columnar (or pin-shaped) convex portion 178, and the connection connector 170 is provided with a concave portion 179 into which the convex portion 178 is fittable. In addition, the concave portion may be provided on the gas supply device 66 side, and the convex portion may be provided on the connection connector 170 side. This allows the convex portion 178 to be coupled to the concave portion 179 in a fitted state when the connection connector 170 of the gas supply pipe 64 is coupled to the automatic gas supply connector 144, whereby the movement of the connection connector 170 in its rotational direction is regulated, and alignment is performed such that the end faces of the side face light leakage type optical fibers 176 and the LEDs 140 face each other. In addition, the position regulating means is not particularly limited if the position regulating means can determine the connection position (position in the rotational direction) of the connection connector 170 such that the end faces of the side face light leakage type optical fibers 176 and the LEDs 140 face each other.

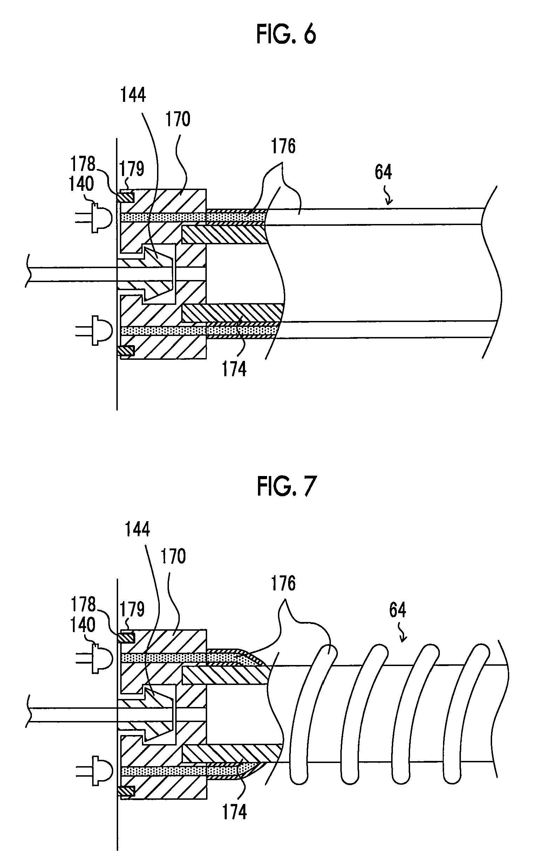

In addition, although the present embodiment has the configuration in which the side face light leakage type optical fibers 176 are buried in the side wall portion of the transparent tube 174, the invention is not limited to this. For example, as shown in Fig. 6, the side face light leakage type optical fibers 176 may be provided along the axial direction at an outer peripheral portion of the transparent tube 174. Additionally, as shown in Fig. 7, the side face light leakage type optical fibers 176 may be provided at the outer peripheral portion of the transparent tube 174 in a state where the optical fibers are spirally wound. According to the configuration in which the side face light leakage type optical fibers 176 are externally attached to the outside of the transparent tube 174 in this way, it is possible to use an opaque tube instead of the transparent tube 174.

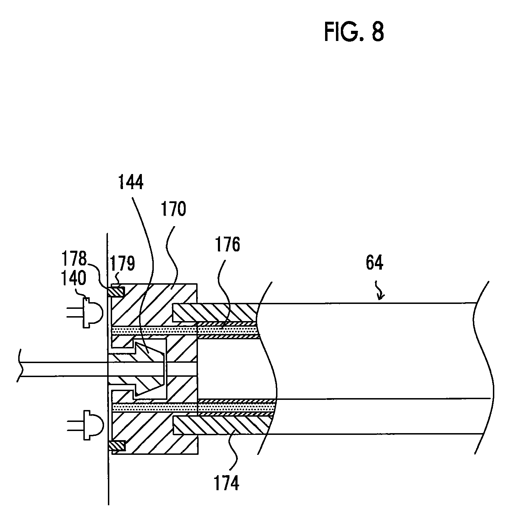

Additionally, as shown in Fig. 8, the side face light leakage type optical fibers 176 may be provided along the axial direction at an inner peripheral portion of the transparent tube 174. Additionally, although illustration is omitted, the side face light leakage type optical fibers 176 may be provided at the inner peripheral portion of the transparent tube 174 in a state where the optical fibers are spirally wound.

Additionally, in the present embodiment, the side face light leakage type optical fibers 176 are used as linear light emitters, however it is also possible to use EL fibers (notifying means) instead of the side face light leakage type optical fibers 176. In this case, when the connection connector 170 of the gas supply pipe 64 is coupled to the automatic gas supply connector 144, EL fibers are configured so as to be electrically connected to the control unit 130. The EL fibers themselves emit light by electroluminescence according to a control signal output from the control unit 130. Accordingly, it is unnecessary to provide the gas supply device 66 with the LEDs 140 in the case of an aspect using the EL fibers.

Additionally, in the present embodiment the gas supply pipe 64 may be constituted of light guide tubes. According to this aspect, since the light guide tubes themselves emits light, it is necessary to provide neither the side face light leakage type optical fibers 176 nor the EL fibers.

Next, the operation of the present embodiment will be described.

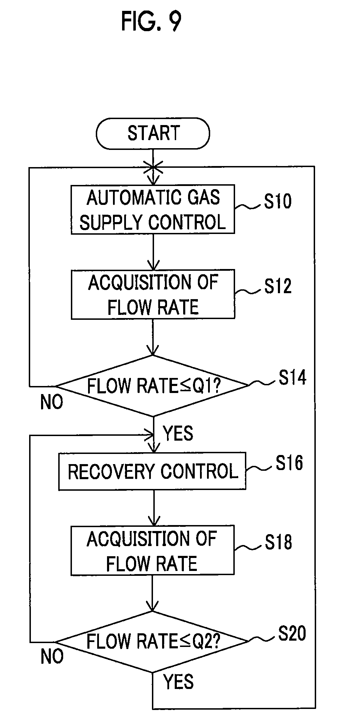

First, if the insertion section 12 of the endoscope 10 is inserted into a body cavity (for example, the stomach, the large intestine, or the like) and the power switch 138 of the gas supply device 66 is turned on, the gas supply device 66 is brought into an operation state. At this time, the control unit 130 detects that the power switch 138 has been turned on, and executes each processing according to a flowchart shown in Fig. 9.

First, the control unit 130 performs automatic gas supply control (Step S10). At this time, the control unit 130 brings the first electromagnetic valve 120 into an opened state and brings the second electromagnetic valve 122 into a closed state. This allows the carbon dioxide gas supplied from the carbon dioxide cylinder 110 to be gradually pressure-reduced to suitable pressure by the first and the second regulators 116 and 118 and be supplied to the automatic gas supply connector 144 via the first electromagnetic valve 120 and the flow rate sensor 124. At this time, the control unit 130 adjusts the flow rate of the carbon dioxide gas while controlling the opening and closing of the first electromagnetic valve 120, on the basis of the pressure (body cavity pressure) within the body cavity detected by the second pressure sensor 128, thereby performing control such that the inside of the body cavity has a set pressure.

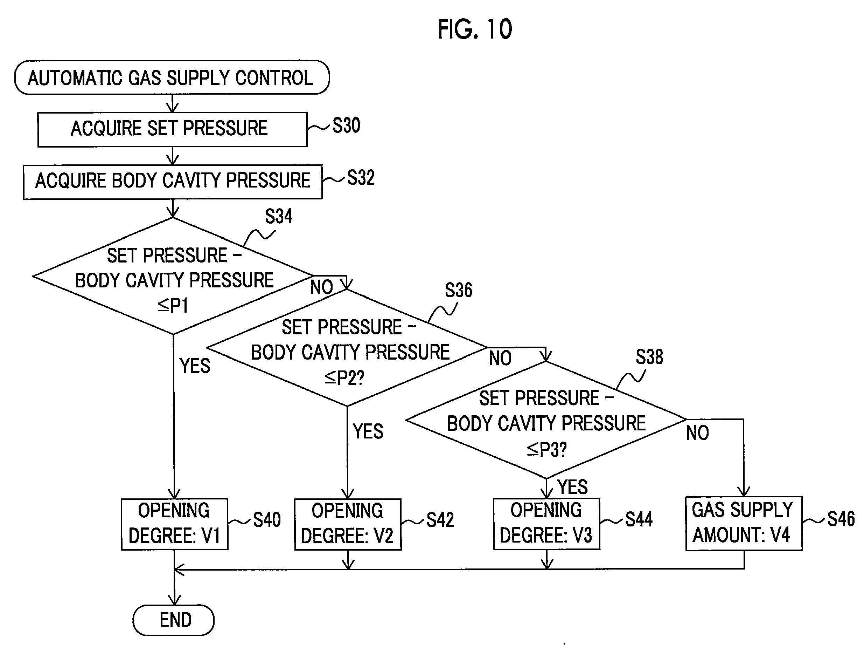

Fig. 10 is a flowchart view showing an example of the automatic gas supply control shown in Fig. 9. Here, a flow rate control valve capable of changing an opening degree (flow channel area) in proportion to a control signal (current value) output from the control unit 130 is used as the first electromagnetic valve 120.

First, the control unit 130 acquires the newest set pressure from the memory (Step S30), and subsequently acquires the pressure (body cavity pressure) within the body cavity detected by the second pressure sensor 128 (Step S32).

Next, the control unit 130 changes the opening degree of the first electromagnetic valve 120 according to the pressure differential between the set pressure and the body cavity pressure (Steps S34 to S46). Here, the opening degree of the first electromagnetic valve 120 is set within a range of V1 to V4 (where 0 < V1 < V2 < V3 < V4) by sequentially comparing the magnitude of the pressure differential between the set pressure and the magnitude of the body cavity pressure with first to third pressure-differential thresholds P1 to P3 (here, 0 < P1 < P2 < P3).

That is, when the pressure differential between the set pressure and the body cavity pressure is equal to or smaller than the first pressure-differential threshold P1 (here, P1 > 0) (Yes in Step S34), the control unit 130 makes the opening degree of the first electromagnetic valve 120 V1 (for example, 25%) (Step S40). In addition, when the body cavity pressure has exceeded the set pressure, the first electromagnetic valve 120 may be brought into a closed state (the opening degree of 0%). However, if the carbon dioxide gas absorbed into the living body is taken into consideration, it is preferable to set the opening degree of the first electromagnetic valve 120 to the opened state within a range that does not exceed V1.

Additionally, when the pressure differential between the set pressure and the body cavity pressure is larger than the first pressure-differential threshold P1 and equal to or smaller than the second pressure-differential threshold P2 (Yes in Step S36), the opening degree of the first electromagnetic valve 120 is made to be V2 (for example, 50%) (Step S42).

Additionally, when the pressure differential between the set pressure and the body cavity pressure is larger than the second pressure-differential threshold P2 and equal to or smaller than the third pressure-differential threshold P3 (Yes in Step S38), the opening degree of the first electromagnetic valve 120 is made to be V3 (for example, 75%) (Step S44).

Additionally, when the pressure differential between the set pressure and the body cavity pressure is larger than the third pressure-differential threshold P3 (No in Step S38), the opening degree of the first electromagnetic valve 120 is made to be V4 (for example, 100%) (Step S46).

According to the automatic gas supply control shown in Fig. 10, the gas supply of carbon dioxide gas is performed such that the pressure within a body cavity becomes a set pressure, and control is performed such that the gas supply amount of the carbon dioxide gas decreases gradually, as the body cavity pressure approaches the set pressure. That is, if the pressure differential between the body cavity pressure and the set pressure is large (for example, immediately after the start of the gas supply of the carbon dioxide gas), the gas supply amount increases, and if the pressure differential between the body cavity pressure and the set pressure becomes small, the gas supply amount decreases. Accordingly, it is possible to suppress a phenomenon in which the body cavity pressure fluctuates before and after the set pressure is reached, and it is possible to make the pressure within a body cavity the set pressure efficiently in a short time.

In addition, in the automatic gas supply control shown in Fig. 10, the gas supply amount of the carbon dioxide gas is changed by changing the opening degree of the first electromagnetic valve 120. However, the invention is not limited to this, and the open time of the first electromagnetic valve 120 may be changed by making the opening degree of the first electromagnetic valve 120 constant, and these may be combined.

Returning to Fig. 9, the processing after the automatic gas supply control in Step S10 is executed will be described. First, the control unit 130 acquires a flow rate (detected flow rate) detected by the flow rate sensor 124 (Step S12).

Next, whether or not the detected flow rate acquired in Step S12 is equal to or smaller than a first flow rate threshold Q1 is determined (Step S14). When the detected flow rate is larger than the first flow rate threshold Q1 (No in Step S14), a normal state is determined. Thus, the processing returns to Step S10 where the automatic gas supply control continues to be executed.

On the other hand, when the detected flow rate is equal to or smaller than the first flow rate threshold Q1 (Yes in Step S14), it is determined that clogging has occurred in a gas supply conduit (gas tube 104 in the present example) serving as a supply path for carbon dioxide gas, and the detected flow rate has declined. Therefore, the control unit 130 executes recovery control (Step S16). At this time, the control unit 130 brings the first electromagnetic valve 120 into a closed state and brings the second electromagnetic valve 122 into an opened state. This allows the carbon dioxide gas pressure-reduced by the first regulator 116 to be supplied to the automatic gas supply connector 144 via the bypass conduit 142. That is, since carbon dioxide gas of higher pressure is supplied to the gas supply conduit compared to the case where the automatic gas supply control is executed, foreign matter, or the like, which is added to the gas supply conduit, is forcedly discharged from the gas jet port 62, and it is possible to resolve the clogging state of the gas supply conduit.

Next, the control unit 130 acquires the flow rate (detected flow rate) detected by the flow rate sensor 124 (Step S18). Then, the control unit 130 determines whether or not the acquired detected flow rate is larger than a second flow rate threshold Q2 (here, Q2 > Q1) (Step S20). When the detected flow rate is equal to or smaller than the second flow rate threshold Q2 (No in Step S20), the clogging of the gas supply conduit is not sufficiently resolved, and there is a possibility the gas supply conduit has become clogged again. Therefore, the processing returns to Step S16 wherein the recovery control continues to be executed until the detected flow rate becomes larger than the second flow rate threshold Q2.

On the other hand, when the detected flow rate is larger than the second flow rate threshold Q2 (Yes in Step S20), it is determined that the clogging of the gas supply conduit is sufficiently resolved. Therefore, the processing returns to Step S10 where the gas supply in the automatic gas supply control is performed.

In this way, in the present embodiment, whether or not clogging has occurred in the gas supply conduit is determined on the basis of the flow rate detected by the flow rate sensor 124, and when the clogging has occurred in the gas supply conduit, the recovery control for recovering from the clogging is executed.

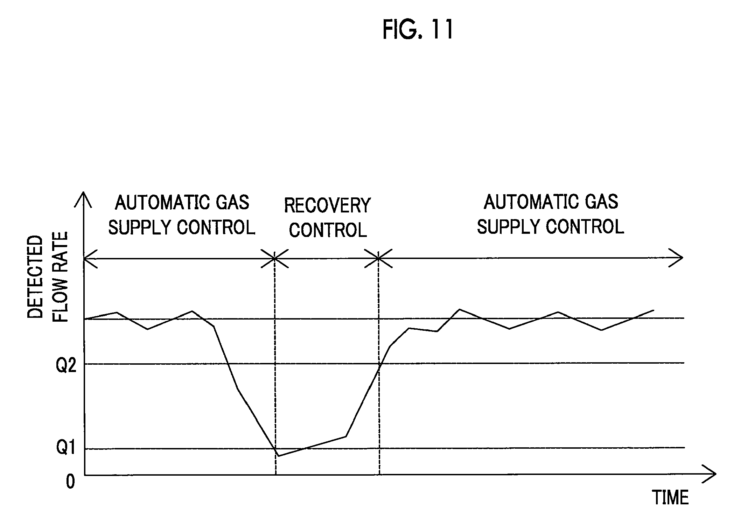

Fig. 11 is a graph schematically showing the state of changes in the detected flow rate before and after the recovery control is executed. As shown in Fig. 11, if clogging occurs in the gas supply conduit when the automatic gas supply control is performed, the detected flow rate declines gradually according to the clogging state.

In the present embodiment, before the gas supply conduit is fully blocked (that is, before the detected flow rate becomes 0), the timing at which the detected flow rate becomes the first flow rate threshold Q1 is detected, and switching from the automatic gas supply control to the recovery control is made. In the recovery control, since carbon dioxide gas of higher pressure is supplied out compared to the automatic gas supply control, and foreign matter added to the gas supply conduit is forcedly discharged from the gas jet port 62.

Additionally, return to the automatic gas supply control is made when the detected flow rate exceeds the first flow rate threshold Q1 after the gas supply in the recovery control is performed for a given period of time, however the foreign matter added to the gas supply conduit may not be sufficiently discharged, clogging may occur again in the gas supply conduit, and the recovery control may be repeatedly performed due to a decline in the detected flow rate.

Thus, in the present embodiment, whether or not the detected flow rate has exceeded the second flow rate threshold Q2 larger than the first flow rate threshold Q1 is determined. If the detected flow rate has exceeded the second flow rate threshold Q2, shift from the recovery control to the automatic gas supply control is made. This can prevent the recovery control from being repeatedly executed and can reliably resolve the clogging that has occurred in the gas supply conduit.

Additionally, in the present embodiment, the control unit 130 performs LED lighting control according to the opened/closed state of the first and second electromagnetic valves 120 and 122.

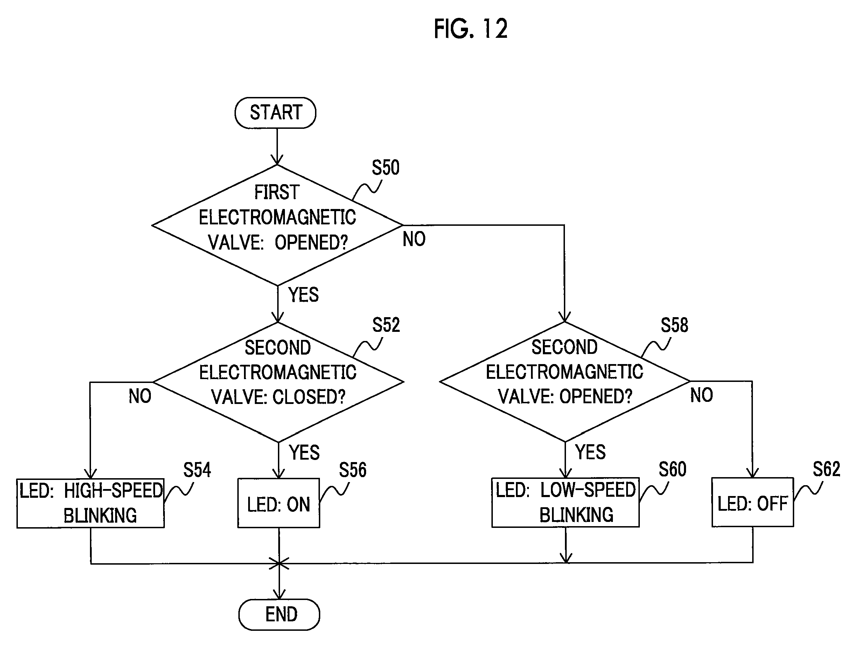

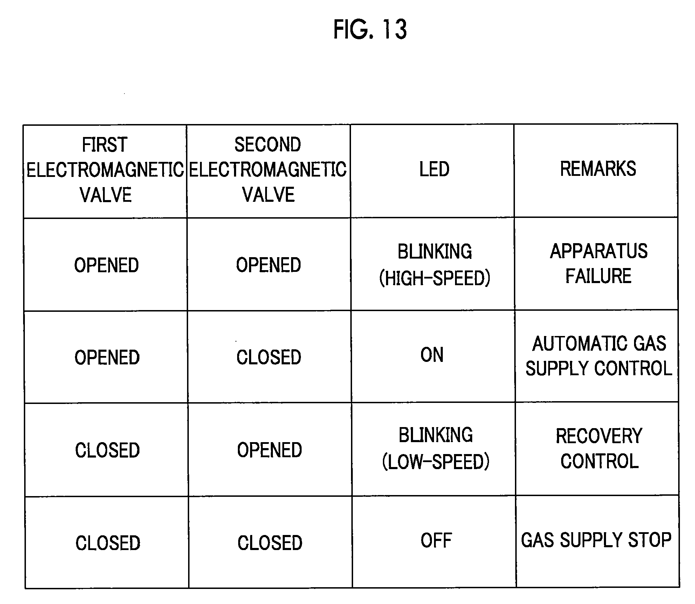

Fig. 12 is a flowchart view showing an example of the LED lighting control. Additionally, Fig. 13 is a view showing the correspondence relationship between the opened/closed state of the first and second electromagnetic valves 120 and 122 and the lighting state of the LEDs 140.

As shown in Fig. 12, the control unit 130 determines the opened/closed state of the first and second electromagnetic valves 120 and 122 according to a predetermined sequence (Steps S50, S52, and S58).

When the first and second electromagnetic valves 120 and 122 are brought into an opened state, the control unit 130 blinks the LEDs 140 at high speed (Step S54). This enables the gas supply pipe 64 to repeat a light-emitting state and a non-light-emitting state in a short cycle fashion and enables the operator to recognize that the apparatus is abnormal.

When the first electromagnetic valve 120 is in an opened state and the second electromagnetic valve 122 is in a closed state, the control unit 130 blinks the LEDs 140 at high speed (Step S56). This enables the gas supply pipe 64 to be brought into an always light-emitting state and enables the operator to recognize that carbon dioxide gas is supplied in the automatic gas supply control.

When the first electromagnetic valve 120 is in a closed state and the second electromagnetic valve 122 is in an opened state, the control unit 130 blinks the LEDs 140 at low speed (Step S60). This enables the gas supply pipe 64 to repeat a light-emitting state and a non-light-emitting state in a long cycle fashion and enables the operator to recognize that carbon dioxide gas is supplied in the recovery control.

When the first and second electromagnetic valves 120 and 122 are in a closed state, the control unit 130 turns off the LEDs 140 (Step S62). This enables the gas supply pipe 64 to be brought into a non-light-emitting state and enables the operator to recognize that gas supply of carbon dioxide gas is stopped.

In this way, according to the present embodiment, the light-emitting state of the gas supply pipe 64 changes according to the opened/closed state of the first and second electromagnetic valves 120 and 122. Thus, when the operation of the endoscope 10 is performed, the operator can easily grasp the supply state of carbon dioxide gas without turning his/her eyes.

In addition, the four LEDs 140 are not limited to LEDs with the same color, and may be used as a combination of LEDs with different colors. For example, the LEDs may be a combination of two green LEDs and two red LEDs.

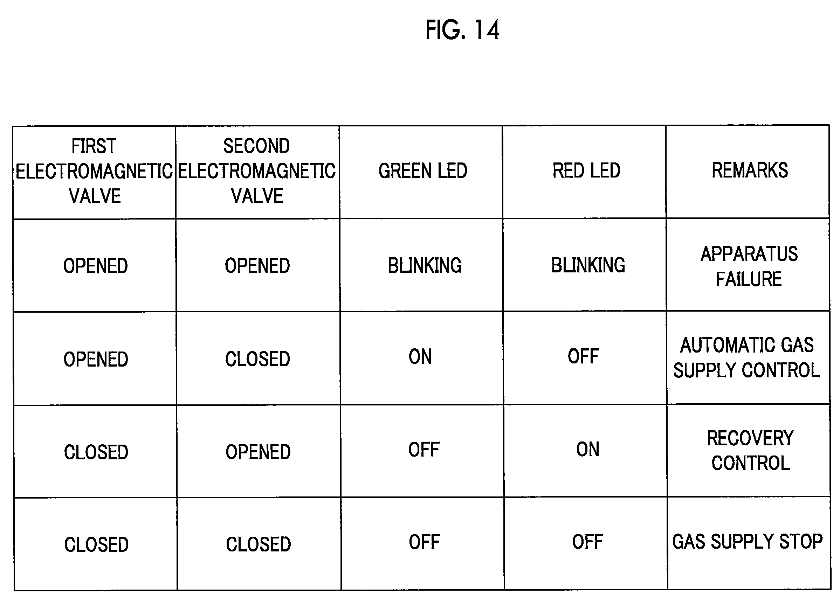

Fig. 14 is a view showing an example of the LED lighting control when LEDs with a plurality of colors are used. In the LED lighting control shown in Fig. 14, both the green LEDs and the red LEDs are brought into a blinking state when both the first and second electromagnetic valves 120 and 122 are in an opened state. Additionally, when the first electromagnetic valve 120 is in an opened state and the second electromagnetic valve is in a closed state, the green LEDs are turned on and the red LEDs are turned off. Additionally, when the first electromagnetic valve 120 is in a closed state and the second electromagnetic valve is in an opened state, the green LEDs are turned off and the red LEDs are turned on. Additionally, when both the first and second electromagnetic valves 120 and 122 are in a closed state, both the green LEDs and the red LEDs are turned off. This enables the operator to easily grasp whether or not gas supply of carbon dioxide gas into the body cavity is normally performed from the luminescent color of the gas supply pipe 64.

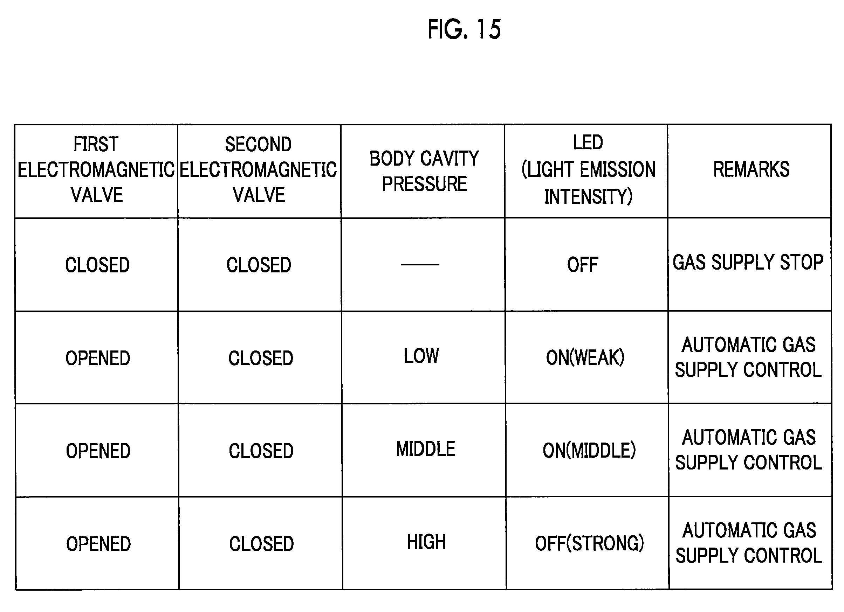

Additionally, in the LED lighting control, the light-emitting state may be changed according to the pressure (body cavity pressure) within the body cavity detected by the second pressure sensor 128. In the example shown in Fig. 15, when the first electromagnetic valve 120 is in an opened state and the second electromagnetic valve 122 is in a closed state, the light emission intensity of the LEDs 140 is gradually changed according to the body cavity pressure when the LEDs 140 are turned on. For example, when the ratio of the body cavity pressure to the set pressure is at a high level (for example, 80% or more), the light emission intensity is defined as strong, when the ratio is at a middle level (for example, 50% or more and less than 80%), the light emission intensity is defined as middle, and when the ratio is at a low level (for example, less than 50%), the light emission intensity is defined as weak. This enables the operator to recognize the state of the body cavity pressure according to the light-emitting state (brightness) of the gas supply pipe 64. In addition, although a description is omitted, the light-emitting state of the LEDs with different colors may be changed according to the state of the body cavity pressure.

As described above, according to the first embodiment, when gas supply of carbon dioxide gas is performed such that the pressure within the body cavity becomes the set pressure, high-pressure carbon dioxide gas is supplied to the gas supply conduit in a case where clogging has occurred in the gas supply conduit. Thus, the clogging of the gas supply conduit can be automatically resolved. This enables carbon dioxide gas to be stably supplied into the body cavity and enables a visual field or a region within the body cavity to be secured. Additionally, since complicated work for resolving the clogging of the gas supply conduit becomes unnecessary, it is possible to reduce an operator's burden.

Additionally, the operator can instantaneously recognize whether or not carbon dioxide gas is being automatically supplied into the body cavity from the gas supply device 66 simply by checking the light-emitting state of the gas supply pipe 64. That is, when the operator is operating the endoscope 10, the operator can easily grasp the supply state of carbon dioxide gas without turning his/her eyes. This can reduce the operator's operation burden and can improve convenience.

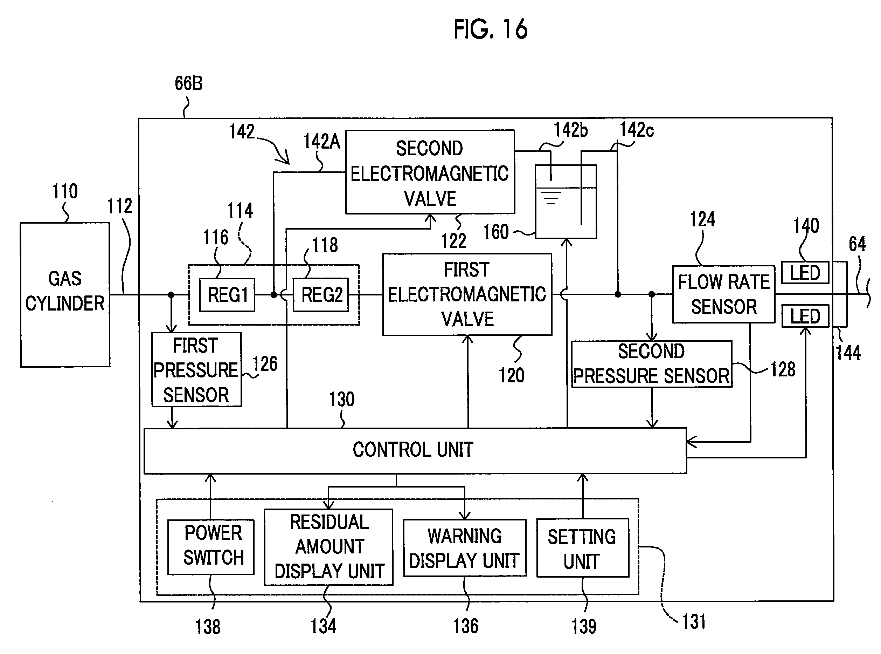

Fig. 16 is a block diagram showing the configuration of a gas supply device 66B. In Fig. 16, members common or similar to those of Fig. 4 will be designated by the same reference numerals, and the description thereof will be omitted.

As shown in Fig. 16, in the gas supply device 66B, the bypass conduit 142 is provided with a liquid supply tank 160. The bypass conduit 142 is constituted of a conduit 142a that connects an outlet side of the first regulator 116 and the second electromagnetic valve 122, a conduit 142b that connects the second electromagnetic valve 122 and the liquid supply tank 160, and a conduit 142c that connects the liquid supply tank 160 and an outlet side of the first electromagnetic valve 120. The conduit 142b communicates with the liquid supply tank 160 above its liquid level, and the conduit 142c communicates with the liquid supply tank 160 below its liquid level. The other configuration is the same as that of the first embodiment.

In the recovery control, if the first electromagnetic valve 120 is brought into a closed state and the second electromagnetic valve 122 is brought into an opened state, the carbon dioxide gas (high pressure gas) pressure-reduced by the first regulator 116 is supplied to above the liquid level of the liquid supply tank 160 via the conduits 142a and 142b. This increases the internal pressure of the liquid supply tank 160 so as to allow water to be supplied to the conduit 142c. Then, water is supplied to the gas supply conduit (gas tube 104) provided in the endoscope 10. Accordingly, even if foreign matter is added to the gas supply conduit and clogging occurs, the foreign matter can be discharged from the gas jet port 62 by the hydraulic pressure related to the gas supply conduit, and it is possible to reliably resolve the clogging of the gas supply conduit.

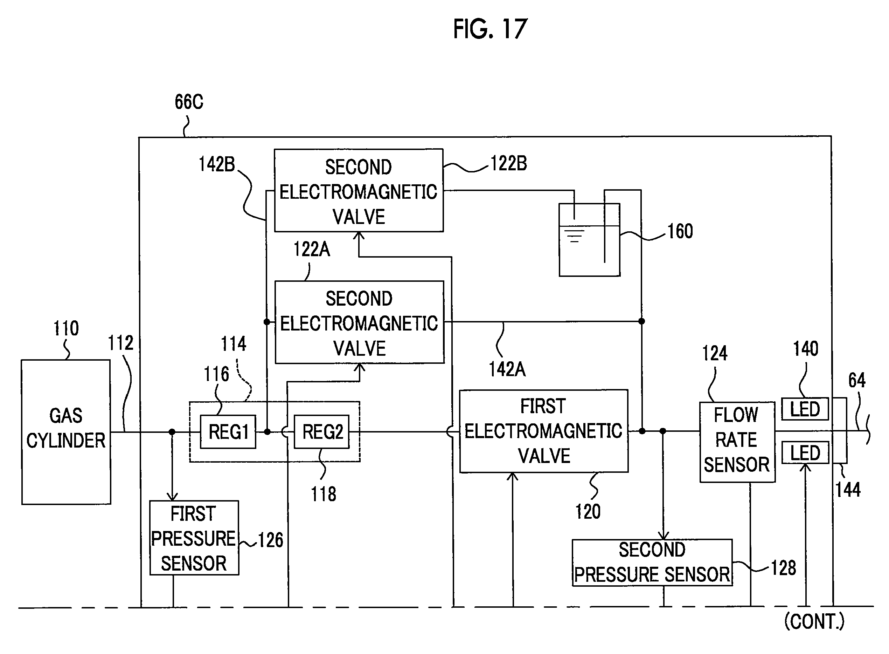

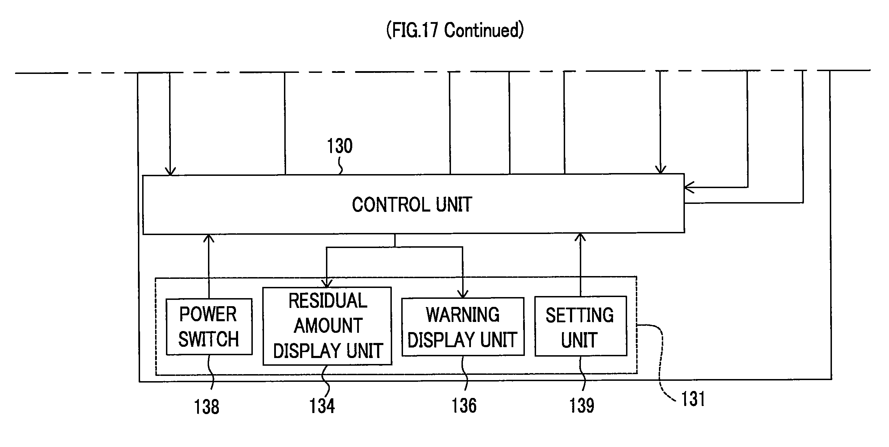

Fig. 17 is a block diagram showing the configuration of a gas supply device 66C.

As shown in Fig. 17, in the gas supply device 66C, a first bypass conduit 142A and a second bypass conduit 142B are connected in parallel to each other, and both ends of each of the bypass conduits 142A and 142B are respectively connected to the same connection positions as the bypass conduit 142 (refer to Fig. 4) of the first embodiment.

Second electromagnetic valves 122A and 122B are disposed at the first and second bypass conduits 142A and 142B, respectively. Additionally, the liquid supply tank 160 as water supply means is provided on an outlet side of the second electromagnetic valve 122B at the second bypass conduit 142B.

In a case where a clogged state has occurred in the gas supply conduit, when the control unit 130 executes the recovery control, it is possible to selectively control the opening and closing of the second electromagnetic valves 122A and 122B, thereby supplying carbon dioxide gas or water to the gas supply conduit. At this time, by bringing the second electromagnetic valve 122B into an opened state so as to supply carbon dioxide gas to the gas supply conduit for a given period of time after the second electromagnetic valve 122A is brought into an opened state so as to supply water to the gas supply conduit for a certain time, the moisture remaining in the gas supply conduit can also be discharged while reliably discharging foreign matter added to the gas supply conduit. This enables carbon dioxide gas to be stably supplied into a body cavity without being influenced by the moisture remaining in the gas supply conduit when the automatic gas supply control is performed after the recovery control is performed.

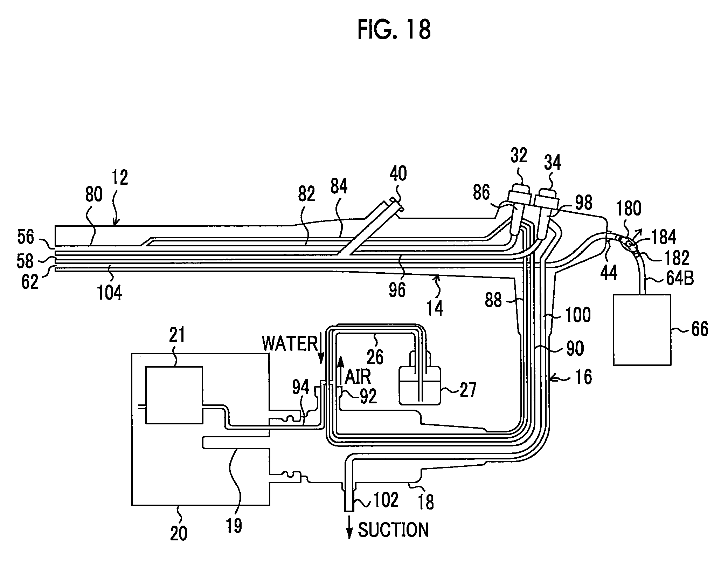

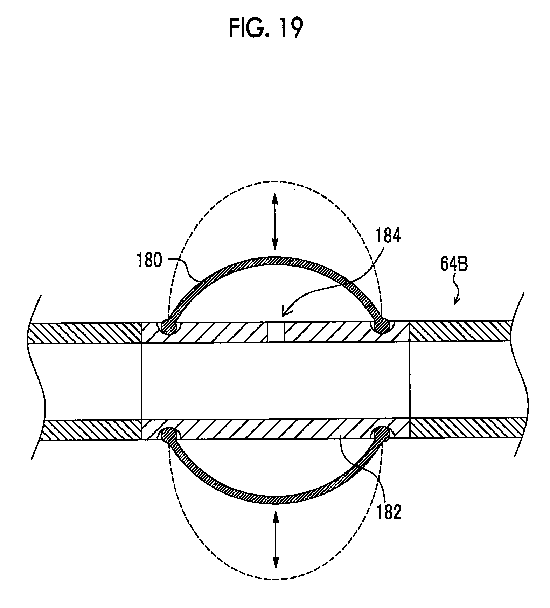

Fig. 18 is a configuration view schematically showing the conduit configuration of an endoscope. Fig. 19 is a schematic cross-sectional view showing essential portions of a gas supply pipe to be used. In Figs. 18 and 19, members common or similar to those of Fig. 3 will be designated by the same reference numerals, and the description thereof will be omitted.

As shown in Figs. 18 and 19, an outer peripheral surface of a gas supply pipe 64B is mounted by a balloon member 180 (notifying means) made of an elastic body, such as rubber. The balloon member 180 is formed in a substantially tubular shape that is constricted at both ends thereof, and is mounted by the gas supply pipe 64B being inserted therethrough and arranged at a desired position and both ends of the balloon member 180 being fixed to a rigid portion (rigid ring) 182 that constitutes a portion of the gas supply pipe 64B. The balloon member 180 is inflatably configured, and is inflated in a substantially spherical shape or deflated and stuck on an outer peripheral surface of the gas supply pipe 64B. In addition, the thickness or material of the balloon member 180 may be appropriately selected such that the inflation amount of the balloon member 180 falls within a suitable range.

An outer peripheral surface of the rigid portion 182 that becomes a mounting position of the balloon member 180 is formed with an opening portion 184. The opening portion 184 communicates with an inner peripheral surface of the gas supply pipe 64B.

While carbon dioxide gas is automatically supplied into a body cavity from the gas supply device 66, the balloon member 180 mounted on an outer peripheral surface of the gas supply pipe 64B serving as the gas supply conduit is brought into an inflation state according to the pressure within the body cavity. This enables an operator to easily grasp that carbon dioxide gas is automatically supplied such that the pressure within the body cavity reaches a desired state without turning his/her eyes even if the operation of the endoscope 10 is being performed.

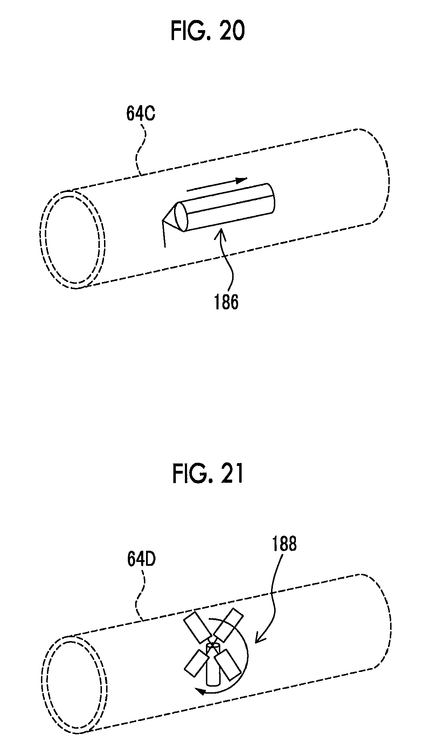

Figs. 20 and 21 are schematic views showing other configuration examples of the gas supply pipe 64. A gas flow detecting member 186 (notifying means) formed in the shape of a streamer (or in the shape of a long flag) that flutters in the flow direction of gas is provided inside a gas supply pipe 64C shown in Fig. 20. Additionally, a gas flow detecting member 188 (notifying means) formed in the shape of a windmill (or in the shape of a propeller) that is rotatably configured according to the flow of gas is provided inside the gas supply pipe 64D shown in Fig. 21. Both of the gas supply pipes 64C and 64D shown in Figs. 20 and 21 are made of transparent tubes (or translucent tubes), and the state of the gas flow detecting member 186 and 188 arranged inside the gas supply pipes can be visually recognized from the outside. This enables an operator to easily grasp that carbon dioxide gas is automatically supplied such that the pressure within the body cavity reaches a desired state without turning his/her eyes even if the operation of the endoscope 10 is performed.

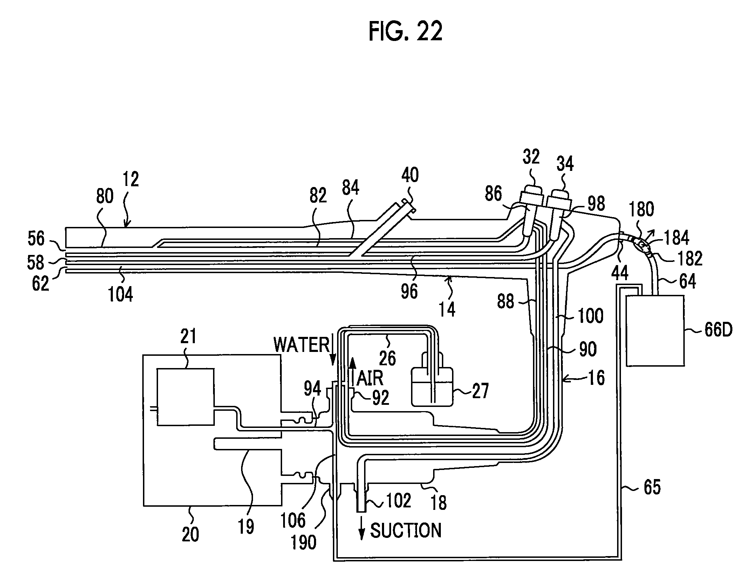

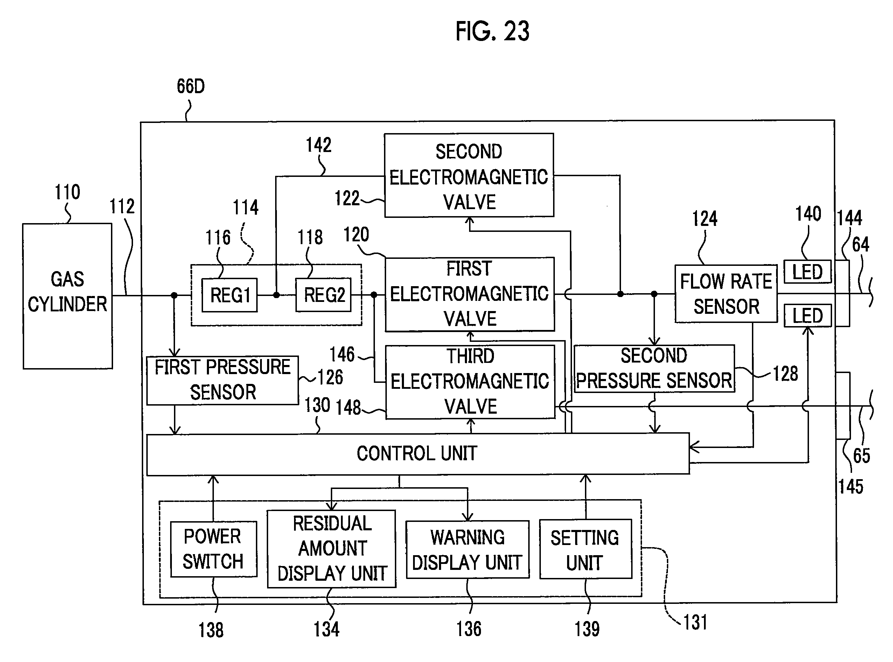

Fig. 22 is a configuration view schematically showing the conduit configuration of an endoscope. Fig. 23 is a block diagram showing the internal configuration of a gas supply device 66D. In Figs. 22 and 23, members common or similar to those of Figs. 3 and 4 will be designated by the same reference numerals, and the description thereof will be omitted.

As shown in Fig. 22, the LG connector 18 is provided with a gas connector 190. One end of the gas supply pipe 65 is detachably connected to the gas connector 190, and the other end of the gas supply pipe 65 is coupled to the gas supply device 66D. Inside the LG connector 18, one end of a gas tube 106 is connected to the gas connector 190, and the other end of the gas tube 106 communicates with the air tube 94.

On the other hand, as shown in Fig. 23, the gas supply device 66D is provided with a manual gas supply connector 145 to which the other end of the gas supply pipe 65 is coupled. Additionally, inside the gas supply device 66D, one end of an internal conduit 146 is connected between the second regulator 118 and the first electromagnetic valve 120, and the other end of the internal conduit 146 is connected to the manual gas supply connector 145. A third electromagnetic valve 148 that operates to open and close on the basis of a control signal output from the control unit 130 is disposed in the internal conduit 146.

The control unit 130 brings the third electromagnetic valve 148 into an opened state, whereby the carbon dioxide gas pressure-reduced by the pressure-reducing mechanism 114 is supplied to the air supply tube 88 via the manual gas supply connector 145 and the gas supply pipe 65, the gas connector 190, the gas tube 106, and the air tube 94. Accordingly, if an operator operates the gas and water supply button 32 similar to a case where air is supplied to the air supply tube 88 from the air pump 21 of the light source device 20, water or carbon dioxide gas is jetted from the gas and water supply nozzle 56.

By operating the gas and water supply button 32 according to a situation, the enables the operator to supply carbon dioxide gas into a body cavity and enables the inside of the body cavity to be finely adjusted to a desired pressure. Accordingly, the inside of the body cavity can be maintained to be in an always suitable state.

In addition, when carbon dioxide gas is supplied from the manual gas supply connector 145 of the gas supply device 66D, it is preferable to stop driving of the air pump 21 so that air is not supplied to the air supply tube 88 via the air tube 94. The air pump 21 is used as an extra gas supply source when the residual amount of the carbon dioxide cylinder 110 is exhausted during the operation of the endoscope 10.

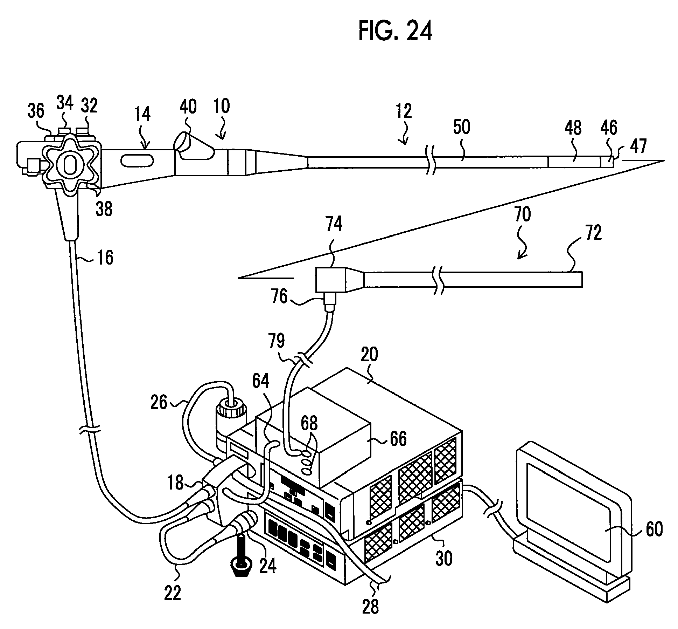

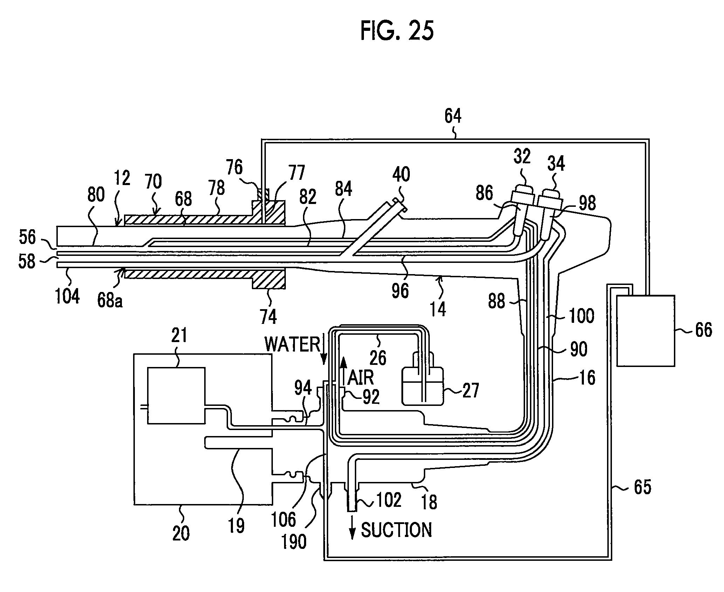

Fig. 24 is an overall configuration view showing the schematic configuration of an endoscope system. Fig. 25 is a conduit configuration view showing the internal configuration of an endoscope shown in Fig. 24. In Figs. 24 and 25, members common or similar to those of Figs. 1 and 3 will be designated by the same reference numerals, and the description thereof will be omitted.

As shown in Figs. 24 and 25, the endoscope system is configured to include an insertion assisting tool 70 through which the insertion section 12 of the endoscope 10 is inserted and guided.

The insertion assisting tool 70 is formed in the shape of a tube, has a slightly larger internal diameter than the external diameter of the insertion section 12, and has sufficient flexibility. A rigid gripping portion 74 is provided at a base end of the insertion assisting tool 70, and the insertion section 12 is inserted from the gripping portion 74.

A gas supply port 76 for supplying carbon dioxide gas is provided in an outer peripheral surface of gripping portion 74. One end of a conduit 77 is connected to the gas supply port 76, and the other end of the conduit 77 opens to an inner peripheral surface of the insertion assisting tool 70, and communicates with an insertion passage 68 formed inside the insertion assisting tool 70.

One end of the gas supply pipe 64 is detachably connected to the gas supply port 76, and the other end of the gas supply pipe 64 is coupled to the gas supply device 66. By supplying carbon dioxide gas from the gas supply device 66, the carbon dioxide gas is supplied from the insertion passage 68 via the gas supply port 44 and the conduit 77, the carbon dioxide gas is introduced from the tip opening portion 68a of the insertion assisting tool 70, and the inside of a body cavity can be swollen with the carbon dioxide gas.

In addition, although illustration is omitted, a valve member is provided as air sealing means that prevents outflow of carbon dioxide gas nearer to the base end side than the opening position of the conduit 77 in the insertion passage 68 of the insertion assisting tool 70. The valve member is formed with a slit hole for allowing the insertion section 12 to be inserted therethrough. Although the shape of the slit hole is not particularly limited, the slit hole is formed, for example, in the shape of a cross. Additionally, from a viewpoint of securement of airtightness, it is preferable that a plurality of valve members be provided at different positions along an axial direction. This enables the carbon dioxide gas supplied from the gas supply device 66 to the insertion passage 68 of the insertion assisting tool 70 to be supplied into a body cavity from the tip opening portion 68a, without flowing out of the base end side.

The insertion passage 68 (specifically, a gap formed between an inner wall surface of the insertion passage 68 and the insertion section 12) formed inside the insertion assisting tool 70 functions as the gas supply conduit for automatically supplying the carbon dioxide gas supplied from the gas supply device 66 into a body cavity. Accordingly, the gas supply conduit for automatically supplying carbon dioxide gas to the endoscope 10 becomes unnecessary. For this reason, it is possible to realize automatic gas supply of carbon dioxide gas even in an endoscope that does not include the gas supply conduit for automatic gas supply.

In addition, in the above-described respective embodiment and examples, the case where flexible scopes, such as an upper gastrointestinal endoscope and a lower gastrointestinal endoscope, are used has been described as an example. However, the gas-supply system of the invention can also be applied to rigid scopes, such as a laparoscope.

Additionally, although the case where carbon dioxide gas is supplied into a body cavity has been described as an example, the gas supplied into the body cavity is not limited to the carbon dioxide gas, and may be other gases, such as helium gas.

Although the gas-supply system related to the invention has been described above in detail, the invention is not limited to the embodiment, and various improvements and modifications may be made without departing from the scope of the invention.

## Claims

1. A gas-supply system that supplies gas into a body cavity of a subject, comprising:
pressure detecting means (128) that detects the pressure within the body cavity;
automatic gas supply means (120, 124) that supplies gas on the basis of the detection result of the pressure detecting means such that the pressure within the body cavity becomes a predetermined pressure;
a gas supply pipe (64) that supplies the gas supplied from the automatic gas supply means into the body cavity; and
notifying means (176, 180, 186, 188) that is provided in the gas supply pipe wherein the notifying means is adapted to visually notify the supply state of the gas by the automatic gas supply means by showing whether or not the supply of the gas by the automatic gas supply means is performed,
**characterized by** control means (130) that outputs a control signal showing whether or not the supply of the gas by the automatic gas supply means is performed,
wherein the notifying means is a light emitter capable of emitting light according to a control signal output from the control means, and
further comprising: a light source (140) capable of emitting light according to a control signal output from the control means,
wherein the light emitter is a side face light leakage type optical fiber on which the light emitted by the light source is incident and that transmits the light while leaking light to a side face.

## Patentansprüche

1. Gaszuführsystem, das Gas in einen Körperhohlraum eines Patienten leitet, umfassend:
eine Drucknachweiseinrichtung (128), die den Druck innerhalb des Körperhohlraums ermittelt;
eine automatische Gaszuführeinrichtung (120, 124), die auf der Grundlage des Nachweisergebnisses von der Drucknachweiseinrichtung Gas derart zuführt, dass der Druck im Inneren des Körperhohlraums zu einem vorbestimmten Druck wird;
eine Gaszuführleitung (64), die das von der automatischen Gaszuführeinrichtung gelieferte Gas in den Körperhohlraum einführt; und
eine Meldeeinrichtung (176, 180, 186, 188), die in der Gaszuführleitung vorgesehen ist, wobei die Meldeeinrichtung dazu ausgebildet ist, den Zuführzustand des Gases durch die automatische Gaszuführeinrichtung dadurch visuell zu melden, dass sie darstellt, ob die Zufuhr des Gases durch die automatische Gaszuführeinrichtung ausgeführt wird oder nicht,
**gekennzeichnet durch**
eine Steuereinrichtung (130), die ein Steuersignal ausgibt, welches angibt, ob die Zufuhr des Gases **durch** die automatische Gaszuführeinrichtung ausgeführt wird oder nicht,
wobei die Meldeeinrichtung ein Lichtabstrahler ist, der in der Lage ist, Licht entsprechend dem von der Steuereinrichtung ausgegebenen Steuersignal abzustrahlen, und
weiterhin umfassend: eine Lichtquelle (140), die in der Lage ist, Licht nach Maßgabe eines von der Steuereinrichtung ausgegebenen Steuersignals zu emittieren,
wobei der Lichtabstrahler eine optische Faser vom Seitenflächen-Lichtaustrittstyp ist, auf die das von der Lichtquelle emittierte Licht auftrifft, und die das Licht bei Ausstrahlen von Licht auf eine Seitenfläche überträgt.

## Revendications

1. Système d'alimentation en gaz alimentant un gaz dans une cavité corporelle d'un sujet, comprenant :
un moyen de détection de pression (128) qui détecte la pression dans la cavité corporelle ;
un moyen d'alimentation en gaz automatique (120, 124) qui alimente le gaz sur la base du résultat de détection du moyen de détection de pression, de sorte que la pression dans la cavité corporelle adopte une pression prédéterminée ;
une conduite d'alimentation en gaz (64) qui alimente le gaz alimenté à partir du moyen d'alimentation en gaz automatique dans la cavité corporelle, et
un moyen de notification (176, 180, 186, 188) qui est fourni dans la conduite d'alimentation en gaz, dans lequel
le moyen de notification est apte à notifier visuellement l'état d'alimentation du gaz par le moyen d'alimentation en gaz automatique en montrant si oui ou non l'alimentation en gaz par le moyen d'alimentation en gaz automatique est réalisée ;
**caractérisé par** un moyen de commande (130) qui produit un signal de commande montrant si oui ou non l'alimentation en gaz par le moyen d'alimentation en gaz automatique est réalisée ;
dans lequel le moyen de notification est un émetteur lumineux en mesure d'émettre une lumière en fonction d'un signal de commande produit à partir du moyen de commande, et
comprenant en outre : une source lumineuse (140) en mesure d'émettre une lumière en fonction d'un signal de commande produit à partir du moyen de commande,
dans lequel l'émetteur lumineux est une fibre optique de type à fuite de lumière de face latérale, sur laquelle la lumière émise par la source lumineuse est incidente et qui transmet la lumière tout en faisant fuir une lumière vers une face latérale.
